# EUROPEAN PATENT SPECIFICATION

(11) **EP 3 357 450 B1**
(45) Date of publication and mention of the grant of the patent: **15.06.2022**
(21) Application number: 18154825.6
(22) Date of filing: 02.02.2018
(51) Int. Cl.: A61C 8/00

(54) **ROTATING ATTACHMENT FOR A DENTAL IMPLANT**
ROTIERENDER AUFSATZ FÜR EIN ZAHNIMPLANTAT
ATTACHEMENT ROTATIF POUR UN IMPLANT DENTAIRE

(30) Priority: 02.02.2017 ES 201730121
(43) Date of publication of application: 08.08.2018
(73) Proprietor: Createch Medical, S.L., 20850 Mendaro (Guipúzcoa) (ES); Bista Eder Clinica Dental, S.L., 20600 Eibar (Guipuzcoa) (ES)
(72) Inventor: URZAINQUI BERISTAIN, Ruben, 20600 EIBAR (Guipúzcoa) (ES)
(74) Representative: Ungria López, Javier

(56) References cited:
- EP-A1- 2 168 531
- EP-A1- 2 486 889
- EP-A1- 2 647 347
- EP-A1- 3 244 825
- ES-U- 1 071 766
- US-A- 5 947 733
- US-A1- 2009 202 962
- US-A1- 2012 315 599

## Description

### Object of the invention

The present invention relates to a rotation dental attachment, which as a result of the capacity thereof to rotate 360° around itself, enables resolving significant implant divergences. The object of the invention is to be applied in dental implantology.

### Technical problem to be solved and background of the invention

Currently, different dental attachments are usually made of one piece and said piece has a fixed inclination (may be 15°, 30°) but none of them can be rotated 360° in order to achieve the desired position and correct any possible implant divergences by means of the attachment.

A dental abutment is known, which has a variable inclination by means of an articulation around which the abutment is articulated and it offers a configurable angle of up to 30°.

Current systems are conditioned by the positions of the anti-rotating elements, which are usually polygons located on the implant, the anti-rotating elements being in charge of establishing the different positions with the sides thereof.

A multitude of documents are known in the state of the art, related to either dental anchoring or dental abutments that offer fixed inclination angles and which require specific and precise previous high precision placement.

Document ES 2264650 A1 discloses a dental implant with rotary crown comprising an implant body formed of at least one anchoring structure, integral with a prosthetic bearing structure and at least one insertion crown for rotation with an outer surface provided with a worm thread, which causes the anchoring structure to be inserted into the bone by screwing the mentioned crown in a bore hole carried out in the bone; wherein the insertion crown of the implant is a unit connected to the anchoring structure such that it is free to rotate with respect thereto and wherein the prosthetic support structure is arranged on the upper end of the anchoring structure, and integral thereto, the insertion crown remaining at the end of said anchoring structure.

Document EP 0874605 B1 discloses a fixture and the prosthesis including said fixture such that the fixture comprises a fastening element of the fixture, which has an insertion end surface and a cylindrical peripheral surface provided with screw threads, and a plurality of circumferentially-spaced cutting recesses, which are formed in a forward, self-tapping end portion of the threaded peripheral surface adjacent to said insertion end surface and which open axially to said insertion end surface, wherein the device has a plurality of tissue collecting and tissue distributing grooves formed in a rear, non-self-tapping portion of the threaded peripheral surface, said grooves opening radially outwards and extending at least partially in the longitudinal direction of the fastening element of the fixture, each groove being, at a forward end thereof, connected and open to one of said cutting recesses for collecting cut-off bone material therefrom and guiding it radially out of the grooves in order to distribute the cut-off bone material circumferentially around the inner wall of the cavity during the insertion of the fixture.

Document US5947733 discloses a connection arrangement between an implant and a straight or angled abutment that can be produced using a clamping screw and an expanding ring. This connection arrangement comprises an inlet in the abutment, and near the lower end of the abutment this inlet is widened via a radially encircling groove. The expanding ring through which the clamping screw passes is supported in the groove, the head of the clamping screw pressing on the expanding ring, and its threaded segment engaging in the threaded bore of the implant. The clamping screw can be pushed from below into the inlet, with the expanding ring pushed on and with the head leading. This document also discloses a special screwdriver is used for screwing.

None of the documents mentioned here above solves the problem of designing an effective and resistant attachment that meets biocompatibility and biomechanical characteristics, suitable for solving the problems of significant divergences of dental implants.

### Description of the invention

The present invention relates to a rotating dental attachment, which facilitates 360 degrees of freedom as opposed to the fixed positions of current systems, allowing the attachment to rotate to an optimal position for the functioning of the attachment itself or for aesthetic purposes.

The rotating dental attachment object of the invention comprises a base piece and an angled top piece wherein the angled top piece is configured to be coupled either by pressure or friction to the base piece, in such a way that there is an existing pressure-friction between the base piece and the angled top piece, as a result of the elasticity of the base piece;
wherein the angled top piece comprises an inner thread that has a longitudinal axis angled with respect to the longitudinal axis of the base piece when said pieces are coupled;
and wherein the angled top piece is also configured to be rotated 360° on the base piece, the base piece being configured to be placed on and fastened onto an implant;
wherein the inner thread is configured for bolting a dental superstructure.

The rotating dental attachment also can comprise:
a screw for fastening the base piece to the implant, wherein said screw is configured to be bolted at an inclined angle from one side of the screw itself and;
an angled wrench, configured for fixing the screw in position from a side of the screw with the necessary inclination, comprising a head designed to be able to bolt the screw onto the implant at the inclination of the attachment.

In the rotating dental attachment object of the invention, the base piece comprises a perimeter ridge and the angled top piece comprises an inner perimeter ridge, such that the inner perimeter ridge of the angled top piece is configured to receive the perimeter ridge of the base piece in order to thus facilitate anchoring of the angled top piece on the base piece.

In the rotating dental attachment object of the invention, the base piece and the angled top piece are configured so that, once coupled together, the screw is caught between the mentioned base piece and the angled top piece.

In an embodiment of the rotating dental attachment object of the invention, the angled top piece comprises an inner thread configured for bolting a dental superstructure.

In another embodiment of the rotating dental attachment object of the invention, the mentioned attachment comprises a matrix which in turn comprises an inner perimeter ridge, furthermore, the angled top piece comprises an outer perimeter ridge, such that the inner perimeter ridge of the matrix is configured to be coupled to the outer perimeter ridge of the angled top piece for anchoring the matrix on the angled top piece.

### Description of the drawings

To complete the description, and for the purpose of helping to make the characteristics of the invention more readily understandable, the present specification is accompanied by a set of drawings constituting an integral part thereof, which by way of illustration and not limitation represent the following:

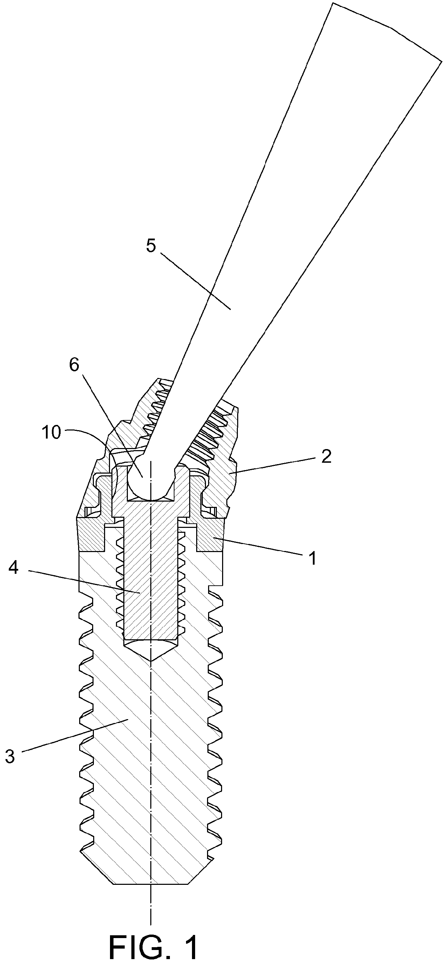

Figure 1 shows a sectional view of the trans-epithelial embodiment of the rotating dental attachment with the angled wrench in bolting position.

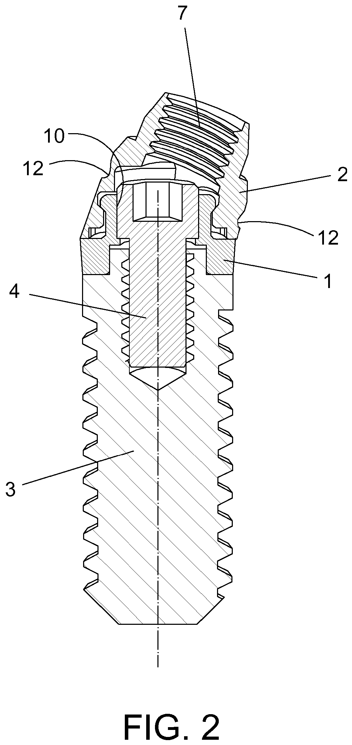

Figure 2 shows a sectional view of the embodiment of the rotating dental attachment of Figure 1 once the angled wrench has been removed.
Figure 3 shows a sectional view of a rotating dental attachment that is not covered by the claimed invention.

Below is a list of the different elements shown in the figures that are included in the invention:
1.- base piece,
2.- angled top piece,
3.- implant,
4.- screw,
5.- angled wrench,
6.- wrench head,
7.- inner thread,
8.- perimeter ridge of the base piece,
9.- inner perimeter ridge of the angled top piece,
10.- inner housing,
11.- matrix
12.- outer perimeter ridge of the angled top piece, and
13.- inner perimeter ridge of the matrix.

### Detailed description of an embodiment of the invention

In view of the foregoing and with reference to the numbering adopted in the figures, the object of the invention is a rotating dental attachment that enables achieving 360° rotation of the attachment itself, thus resolving implant divergences.

The attachment object of the invention comprises a base piece (1) and an angled top piece (2), which has the capacity of rotating 360 degrees on the base piece (1). The base piece (1) is placed on an implant (3) and is fastened to the implant (3) by means of a screw (4), such that the base piece (1) comprises an inner housing (10) for passing the screw (4), while the angled top piece (2) is coupled either by pressure or friction to the base piece (1).

The base piece (1) and the angled top piece (2) are coupled as a result of the elasticity of the base piece (1).

The base piece (1) comprises a perimeter ridge (8) and the angled top piece (2) comprises an inner perimeter ridge (9), such that the inner perimeter ridge (9) of the angled top piece (2) is configured to receive the perimeter ridge (8) of the base piece (1) and thus enable the angled top piece (2) to be anchored on the base piece (1). Likewise, the perimeter ridge (8) and the inner perimeter ridge (9) are in charge of fastening the base piece (1) and the angled top piece (2) together, once the screw (4) is screwed into the implant (3).

The screw (4) used for fastening the base piece (1) and the implant (3) is caught between the base piece (1) and the angled top piece (2) once the angled top piece (2) has been coupled to the base piece (1).

The screw (4) of the attachment object of the invention has an angled head, such that the screw (4) can be bolted at an inclined angle from one side of the screw itself (4). In order to fix the screw (4) in position from a side of the screw (4) with the necessary inclination, the attachment object of the invention has an angled wrench (5) specially developed for the invention. The angled wrench (5) has a head (6) designed to be able to bolt the screw (4) at the inclination of the attachment.

Furthermore, in the attachment object of the invention, the pressure-friction existing between the base piece (1) and the angled top piece (2), allows the attachment object of the invention to be rotated and kept stable in the fixed position by means of the angled wrench (5).

In the attachment object of the invention, the base piece (1), the screw (4) and the angled top piece (2) are factory-assembled for use.

The attachment object of the invention offers the advantage that for the placement thereof in the patient's mouth, it is first bolted into the mouth in any position and subsequently the angled top piece (2) is rotated on the base piece (1) until said angled top piece (2) is placed in the position of interest by using a wrench for this purpose.

The method for fastening the attachment object of the invention is as follows: the base piece (1) is fastened to the implant (3) by screwing the screw (4), but without completely tightening said screw (4), then the angled top piece (2) is positioned in the desired position and the screw (4) is finally tightened.

The final adjustment of the screw (4) against the base piece (1) enables locking the elasticity of the base piece (1) and thereby lock the position of the angled top piece (2) on the base piece (1).

Once the dental attachment object of the invention has been directed to the desired position, it is used as a normal attachment.

The attachment object of the invention is particularly interesting in cases of divergent implants or vestibular implants.

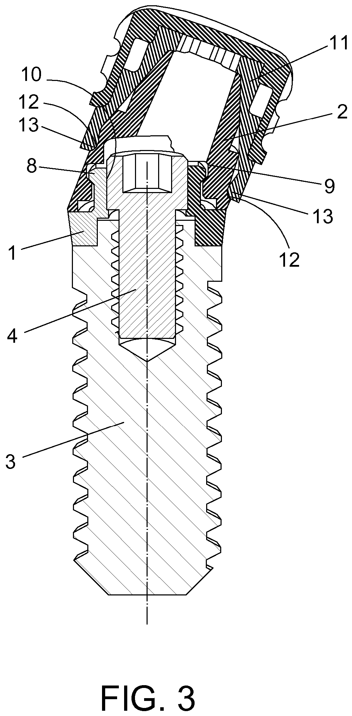

In an embodiment, the attachment object of the claimed invention is applied for a rotating trans-epithelial attachment: the attachment is bolted onto the implant (3) and can be used for bolting a dental superstructure. In this case, the attachment allows the angled top piece (2) to be rotated 360 degrees until reaching an optimal position for exit of the screw (4) in the required area. In this embodiment of the attachment, the angled top piece (2) comprises an inner thread (7) wherein the dental superstructure is bolted. The attachment shown on Figure 3 is bolted onto the implant (3) and can be used to anchor a matrix (11) which in turn is fastened to a removable prosthesis. In this case, the attachment allows the angled top piece (2) to be rotated 360 degrees until achieving a common insertion axis with the remaining anchorages. In this example of attachment, the angled top piece (2) comprises an outer perimeter ridge (12), which is complemented by an inner perimeter ridge (13) of the matrix (11) for anchoring the matrix (11) on the angled top piece (2).

The invention is not intended to be limited to the specific embodiment described in this document. Those skilled in the art may develop other embodiments in light of the description made herein. As such, the scope of the invention is defined by the following claims.

## Claims

1. A rotating dental attachment comprising a base piece (1) and an angled top piece (2)
**wherein** the angled top piece (2) is configured to be coupled either by pressure or friction to the base piece (1), in such a way that there is an existing pressure-friction between the base piece (1) and the angled top piece (2), as a result of the elasticity of the base piece (1);
wherein the angled top piece (2) comprises an inner thread (7) that has a longitudinal axis angled with respect to the longitudinal axis of the base piece (1) when said pieces are coupled;
wherein the angled top piece (2) is also configured to be rotated 360° on the base piece (1), the base piece (1) being configured to be placed on and fastened onto an implant (3); and
wherein the inner thread (7) is configured for bolting a dental superstructure.

2. The rotating dental attachment according to claim 1, **characterized in that** it comprises:
- a screw (4) for fastening the base piece (1) to the implant (3), wherein said screw (4) is configured to be bolted at an inclined angle from one side of the screw itself (4) and;
- an angled wrench (5), configured for fixing the screw (4) in position from a side of the screw (4) with the necessary inclination, comprising a head (6) designed to be able to bolt the screw (4) onto the implant (3) at the inclination of the attachment.

3. The rotating dental attachment according to any of the preceding claims, **characterized in that**
- the base piece (1) comprises a perimeter ridge (8) and
- the angled top piece (2) comprises an inner perimeter ridge (9)
such that the inner perimeter ridge (9) of the angled top piece (2) is configured to receive the perimeter ridge (8) of the base piece (1) for anchoring the angled top piece (2) on the base piece (1).

4. The rotating dental attachment according to claim 2, **characterized in that** the base piece (1) comprises an inner housing (10) for passing the screw (4), wherein the base piece (1) and the angled top piece (2) are configured so that, once coupled together, the screw (4) is caught between the mentioned base piece (1) and the angled top piece (2).

## Patentansprüche

1. Rotierende Dentalbefestigung, umfassend ein Basisstück (1) und ein abgewinkeltes oberes Stück (2),
wobei das abgewinkelte obere Stück (2) so ausgestaltet ist, dass es entweder durch Druck oder Reibung mit dem Basisstück (1) gekoppelt ist, so dass eine Druck-Reibung zwischen dem Basisstück (1) und dem abgewinkelten oberen Stück (2) aufgrund der Elastizität des Basisstücks (1) besteht;
wobei das abgewinkelte obere Stück (2) ein Innengewinde (7) umfasst, das eine Längsachse aufweist, die in Bezug auf die Längsachse des Basisstücks (1) abgewinkelt ist, wenn die Stücke gekoppelt sind;
wobei das abgewinkelte obere Stück (2) außerdem so ausgestaltet ist, dass es um 360° auf dem Basisstück (1) gedreht werden kann, wobei das Basisstück (1) so ausgestattet ist, dass es auf ein Implantat (3) aufgesetzt und daran befestigt werden kann; und
wobei das Innengewinde (7) zur Verschraubung einer dentalen Superstruktur ausgestaltet ist.

2. Rotierende Dentalbefestigung nach Anspruch 1, **dadurch gekennzeichnet, dass** sie umfasst:
- eine Schraube (4) zum Befestigen des Basisstücks (1) an dem Implantat (3), wobei die Schraube (4) so ausgestaltet ist, dass sie in einem geneigten Winkel von einer Seite der Schraube selbst (4) aus verschraubt werden kann und;
- einen abgewinkelten Schraubenschlüssel (5), der so ausgestaltet ist, dass er die Schraube (4) von einer Seite der Schraube (4) mit der erforderlichen Neigung aus fixiert, und der einen Kopf (6) umfasst, der so ausgestaltet ist, dass er die Schraube (4) auf dem Implantat (3) mit der Neigung der Befestigung festschrauben kann.

3. Rotierende Dentalbefestigung nach einem der vorhergehenden Ansprüche, **dadurch gekennzeichnet, dass**
- das Basisstück (1) einen umlaufenden Steg (8) umfasst und
- das abgewinkelte obere Stück (2) einen inneren umlaufenden Steg (9) umfasst, so dass der innere umlaufende Steg (9) des abgewinkelten oberen Stücks (2) so ausgestaltet ist, dass er den umlaufenden Steg (8) des Basisstücks (1) aufnimmt, um das abgewinkelte obere Stück (2) auf dem Basisstück (1) zu verankern.

4. Rotierende Dentalbefestigung nach Anspruch 2, **dadurch gekennzeichnet, dass** das Basisstück (1) ein inneres Gehäuse (10) zum Durchführen der Schraube (4) umfasst, wobei das Basisstück (1) und das abgewinkelte obere Stück (2) so ausgestaltet sind, dass die Schraube (4), sobald sie miteinander gekoppelt sind, zwischen dem genannten Basisstück (1) und dem abgewinkelten oberen Stück (2) eingeklemmt ist.

## Revendications

1. Une fixation dentaire rotative comprenant une pièce de base (1) et une pièce supérieure angulée (2),
la pièce supérieure angulée (2) étant configurée pour être reliée soit par pression, soit par friction, à la pièce de base (1), de telle manière qu'il y ait un frottement par pression existant entre la pièce de base (1) et la pièce supérieure angulée (2), du fait de l'élasticité de la pièce de base (1) ;
la pièce supérieure angulée (2) comprenant un filetage intérieur (7) qui a un axe longitudinal incliné par rapport à l'axe longitudinal de la pièce de base (1) lorsque lesdites pièces sont reliées ;
la pièce supérieure angulée (2) étant également configurée de façon à être tournée sur 360° sur la pièce de base (1), la pièce de base (1) étant configurée de façon à être placée sur un implant (3) et fixée à celui-ci ; et
le filetage intérieur (7) étant configuré pour boulonner une superstructure dentaire.

2. La fixation dentaire rotative selon la revendication 1, **caractérisée en ce qu'**elle comprend :
- une vis (4) pour fixer la pièce de base (1) à l'implant (3), ladite vis (4) étant configurée de façon à être boulonnée selon un angle incliné depuis un côté de la vis (4) elle-même, et
- une clé angulée (5), configurée pour fixer la vis (4) en position depuis un côté de la vis (4) avec l'inclinaison nécessaire, comprenant une tête (6) conçue de façon à être apte à boulonner la vis (4) sur l'implant (3) à l'inclinaison de la fixation.

3. La fixation dentaire rotative selon l'une quelconque des revendications précédentes, **caractérisée en ce que**
- la pièce de base (1) comprend une nervure périphérique (8) et
- la pièce supérieure angulée (2) comprend une nervure périmétrique intérieure (9)
de sorte que l'arête périmétrique intérieure (9) de la pièce supérieure angulée (2) est configurée pour recevoir l'arête périmétrique (8) de la pièce de base (1) pour ancrer la pièce supérieure angulée (2) sur la pièce de base (1).

4. La fixation dentaire rotative selon la revendication 2, **caractérisée en ce que** la pièce de base (1) comporte un logement intérieur (10) de passage de la vis (4), la pièce de base (1) et la pièce supérieure angulée (2) étant configurées de telle sorte qu'une fois reliées l'une à l'autre, la vis (4) est prise entre la pièce de base (1) mentionnée et la pièce supérieure angulée (2).
